# EUROPEAN PATENT SPECIFICATION

(11) **EP 2 439 287 B1**
(45) Date of publication and mention of the grant of the patent: **17.04.2013**
(21) Application number: 11425226.5
(22) Date of filing: 26.08.2011
(51) Int. Cl.: C14B 1/40, G01N 3/34, G01N 33/44, G01N 3/08

(54) **Method for evaluating the efficiency and/or operating state of a staking machine and industrial kit suitable to implement such a method**
Methode zur Bewertung der Effizienz und / oder Betriebszustand einer Stollmaschine und Industrie-Kit geeignet, ein solches Verfahren zu implementieren
Méthode d'évaluation de l'efficacité et / ou l'état de fonctionnement d'une machine à palissonner et kit industriel approprié à mettre en ouvre une telle méthode

(30) Priority: 06.10.2010 IT VI20100271
(43) Date of publication of application: 11.04.2012
(73) Proprietor: Equitan S.r.l., 36072 Chiampo (VI) (IT)
(72) Inventor: Antoniazzi, Antonio, 36072 Chiampo (VI) (IT)
(74) Representative: Santi, Filippo

(56) References cited:
- EP-A2- 1 081 235
- WO-A1-00/68438
- WO-A1-2008/101825
- FR-A- 1 275 070
- US-A- 4 100 771

## Description

The present invention refers to a method for evaluating the efficiency and/or operating state of a staking machine (also known more simply as staker), especially for tanning field (see for example WO 2008/101825 and WO 00/68438).

The invention described herein also relates to an industrial kit suitable to implement such evaluating method.

As known, in particular in the field of leather tanning, staking machines (or stakers) are used to perform a mechanical operation, exactly called staking, before and/or after drying and conditioning phase.

In summary, staking consists in stretching the tanned leather under pressure and/or compression, through appropriate construction tool bodies (for example pins, rollers, smoothed blades or even more) in order to cause the detachment of the dermal fibers hardened and glued together as a result of drying and thereby soften the leather itself.

Performances or action capacity of the staking machines, of course in terms of softening and stretching of the flexible laminar surface (leather) worked by them, are always difficult to measure.

This is directly related to the fact that, as widely known in the tanning industry, different batches of leathers of or even leathers of the same batch which must be subjected to staking often differ from each other by parameters such as thickness, homogeneity and uniformity of the fibres structure, chemical treatment, degree of inherent elasticity, type of subsequent finishing, interfibred content of re-tanning and so on.

The impact of these factors is so high as to prevent, indeed, the determination of the actual efficiency of the staking machine since a lesser degree of softening or reduced level of stretching of the leather can be still attributed to a different value of at least one of the leather parameters just mentioned.

Likewise, the same tool bodies or other mechanical components of the staking machine are sometimes subject over time to wear or malfunctions that affect the efficiency of the staking machine.

At the current state of the art, especially in the tanning field, widely felt and widespread need by the experts therefore persists to measure or evaluate the efficiency of a staking machine in order to optimize working of flexible laminar surface and improve the quality of the finished product.

The present invention intends to overcome the limitations of the prior art just complained.

In particular, primary purpose of the present invention is to implement a method for evaluating the efficiency and/or operating state of a staking machine.

Within such a purpose, it is task of the present invention to improve the efficiency of a staking machine.

It is also task of the present invention to devise a method for evaluating the efficiency and/or operating state of a staking machine that, ultimately, allows to get flexible laminar surfaces, such as leathers, of higher quality than that one currently achievable.

It is another purpose of the invention to provide a method for evaluating the efficiency and/or operating state of a staking machine which is extremely reliable, as much as possible scientific and irrefutable, leaving minimum space to empirical evaluations performed by experts.

It is a last but not least purpose of the present invention to provide a practical industrial kit for evaluating the efficiency and/or operating state of a staking machine.

Said purposes are achieved through a method for evaluating the efficiency and/or operating state of a staking machine, especially for tanning field, as the attached claim 1, as hereinafter referred for the sake of exposure brevity.

Further applicative features of detail of the method of the invention are set forth in the respective dependent claims.

An integral part of the current invention is an industrial kit as the appended claim 13, as hereinafter referred for the sake of exhibition simplicity too.

Further technical features of detail of the industrial kit of the invention are contained in the corresponding dependent claim.

Advantageously, the method of the invention allows to measure in conventional and scientific way, through an evaluation standard represented by the laminar sample sheet, the efficiency of a staking machine, thereby covering a clear lack of the known technique.

Still advantageously, the method of the invention involves the achievement of immediately observable and detectable results possibly reflecting in a timely intervention of adjustment on the operating parameters of the staking machine in order to reach the desired degree of softening and level of stretching in the effective and practical working of the flexible laminar surfaces, such as leathers.

Equally advantageously, the invention allows to improve and ensure a quality constancy of the finished product, at least from the point of view of its softness and its superficial extension which is increased.

In advantageous manner, in addition, the method of the invention allows to perform a comparison between the efficiencies of staking machines distinct each other, built for example by different manufacturers, the laminar sample sheet used for the evaluation test being equal.

Further features and peculiarities of the invention will appear to a greater extent from the description that follows relating to a preferred applicative embodiment of the method and a preferred embodiment of the industrial kit, both hereinafter exclusively claimed, given by illustrative and indicative, but not limiting, way.

According to the invention, the method for evaluating the efficiency and/or operating state of a staking machine, especially for tanning field, includes the following operations:
- introducing a laminar sample sheet, made of deformable material and having predetermined initial dimensions, into a staking machine that:
   ● softens a flexible laminar surface (semi-finished or finished) to be processed, such as leather in tanning industry,
   ● extends or enlarges the flexible laminar surface, increasing the total extension thereof;
- submitting the laminar sample sheet to the vibrating or intermittent action of the tool bodies (for instance pins) of the staking machine, usually while the laminar sample sheet advances along a longitudinal direction hold on a conveyor belt;
- measuring the final dimensions of the laminar sample sheet once it has been subjected to the vibrating or intermittent action of the tool bodies of the staking machine;
- comparing the final dimensions with the initial dimensions of the laminar sample sheet calculating the efficiency of the staking machine by difference between the aforesaid dimensions.

In particular, the deformable material of the laminar sample sheet presents a very low or virtually null elastic memory, where elastic memory refers to the measure of the ability of a polymeric material, such as for instance an elastomer, to return to the initial configuration, once the compressive load applied to it has been removed.

In other words, the laminar sample sheet made of deformable material presents an extremely high or substantially maximum value of permanent traction deformation, considering that, as known, the tool bodies of the staking machine act by traction (or radial extension) on the flexible laminar surface to be worked.

In particular, the maximum elastic return of the laminar sample sheet after being stressed by the tool bodies of the staking machine is less than 10%, preferably equal to 0%.

In addition, the laminar sample sheet made of deformable material has a percentage elongation having a value in the range of 0.3÷600%.

More in detail, the laminar sample sheet made of deformable material has a thickness having a value in the range of 0.013÷13.2 mm, preferably 0.08 mm.

The profile of the laminar sample sheet can take different conformations, according to application needs, although in general it is regular (for instance rectangular, square with 1.OOOx1000 mm. or 3.3x3.3 feet).

According to the preferred applicative embodiment of the method of the invention, the deformable material with which is made the laminar sample sheet is any of the materials capable of plastic deformation selected from the group consisting of polymer, crystalline polymer, tar, lead, mineral bound compound and similar.

Preferably but not necessarily, the crystallized polymer is polytetrafluoroethylene (PTFE acronym, also known under the registered trademark Teflon®), plastic material resistant to high temperatures (up to 200 °C).

The tool bodies of the staking machine perform the vibrating or intermittent action on the laminar sample sheet at a predefined pressure and with a prefixed frequency of strokes per minute: the values of pressure and frequency of strokes per minute with which the tool bodies operate on the laminar sample sheet are the same values used for the actual working of the flexible laminar surface semi-finished or finished (for example the leather) in the staking machine.

Consequently, the predefined pressure and prefixed frequency of strokes per minute vary depending on the type of semi-finished flexible laminar surface to be worked in the staking machine.

In practice, therefore, according to the method of the invention, in the staking machine the laminar sample sheet undergoes the same mechanical operation to which the semi-finished flexible laminar surface is subjected, advancing through it at a predetermined speed until to get out from it immediately after being subjected to the action of the tool bodies whose operation parameters are set according to the values of normal and traditional use of the machine itself.

By strictly preferred but not exclusive title, at the end of a cycle of use, the laminar sample sheet is stored in order to be reusable for at least another separate cycle of evaluation of the efficiency and/or operating state of the staking machine.

In the new and further cycle of evaluation of the efficiency of the staking machine the laminar sample sheet could optionally be subjected to different conditions of penetration (or pressure) and strokes per minute of the tool bodies if the staking machine might, for example, work a flexible laminar surface of different type with respect to the previous one.

Other applicative embodiments of the method of the invention could provide that the laminar sample sheet is introduced into the staking machine along with the flexible laminar surface (semi-finished or finished) to be processed, for example directly overlapping or below or immediately following the flexible laminar surface itself.

As said, object of the present invention is also an industrial kit for the evaluation of the efficiency and/or operating state of a staking machine, especially for tanning field.

In accordance with the invention, the kit industrial includes:
- a staking machine suitable to soften a semi-finished flexible laminar surface to be worked as well as to stretch it;
- a laminar sample sheet, made of deformable material and having a predetermined initial dimensions, suitable to be introduced into the staking machine in order to be subjected, similarly to the flexible laminar surface, to the vibrating or intermittent action of the tool bodies of the staking machine.

Based on the foregoing, it is understood, therefore, that the method for evaluating the efficiency and/or operating state of a staking machine and the industrial kit suitable to implement such a method, both object of the present invention, achieve the purposes and reach the advantages mentioned above.

In execution phase, changes can be made to the method of the invention consisting, for example, in a number of laminar sample sheets introduced into the staking machine in order to assess the relative efficiency greater than one.

In addition, in other solutions, the industrial kit object of the invention may include more than a single laminar sample sheet made of deformable material.

It is stated precisely that, in case of use in tannery field, the stacking machine suitable to implement the method here claimed could be suitable to indifferently treat wet leathers *("wet staking", "wet stretching"* or *"wet perching")* o almost dry leathers *("dry staking"* or *"dry stretching"* or *"dry perching").*

It is, then, clear that many other variations can be made to the method and kit in exam, without for this reason departing from the principle of novelty intrinsic in the inventive idea expressed here, as it is clear that, in the practical implementation of the invention, materials can be changed, as needed, and replaced with others technically equivalent.

## Claims

1. Method for evaluating the efficiency and/or operating state of a staking machine, especially for tanning field, **characterized in that** it comprises the following operations:
- introducing at least one laminar sample sheet made of deformable material and having predetermined initial dimensions, into a staking machine capable of:
• softening at least one flexible laminar surface to be worked,
• stretching or enlarging said flexible laminar surface;
- submitting said laminar sample sheet to the vibrating or intermittent action of the tool bodies of said staking machine;
- measuring the final dimensions of said laminar sample sheet once it has been subjected to said vibrating or intermittent action of said tool bodies of said staking machine;
- comparing said final dimensions with said initial dimensions of said laminar sample sheet calculating the efficiency of said staking machine by difference between said dimensions,
wherein said deformable material of said laminar sample sheet has an extremely low or essentially null elastic memory.

2. Method according to claim 1 **characterized in that** said laminar sample sheet made of deformable material has an extremely high or substantially maximum value of permanent tensile deformation.

3. Method according to any of the preceding claims, **characterized in that** said laminar sample sheet made of deformable material has a thickness having a value in the range of 0.013÷13.2 mm.

4. Method according to any of the preceding claims, **characterized in that** said laminar sample sheet made of deformable material has a percentage elongation having a value in the range of 0.3÷600%.

5. Method according to any of the preceding claims **characterized in that** said deformable material with which said laminar sample sheet is made of is any of the materials capable of plastic deformation selected from the group consisting of polymer, crystalline polymer, tar, lead, mineral bound compound and similar.

6. Method according to claim 5 **characterized in that** said crystalline polymer is polytetrafluoroethylene (PTFE).

7. Method according to any of the preceding claims, **characterized in that** said tool bodies of said staking machine perform said vibrating or intermittent action on said laminar sample sheet at a predefined pressure and with a prefixed frequency of strokes per minute.

8. Method according to claim 7 **characterized in that** said predefined pressure and said prefixed frequency of strokes per minute vary depending on the type of said flexible laminar surface to be worked in said staking machine.

9. Method according to any of the preceding claims, **characterized in that** said laminar sample sheet can be reused in at least one another separate cycle of evaluation of said efficiency of said staking machine.

10. Method according to any of the preceding claims, **characterized in that** said laminar sample sheet is introduced into said staking machine together with said flexible laminar surface to be worked.

11. Kit for the evaluation of the efficiency and/or operating state of a staking machine, especially for tanning sector, **characterized in that** it includes:
- a staking machine suitable to soften at least one flexible laminar surface to be worked and stretch or enlarge said flexible laminar surface;
- at least one laminar sample sheet, made of deformable material and having predetermined initial dimensions, suitable to be introduced into said staking machine in order to be subjected, similarly to said flexible laminar surface, to the vibrating or intermittent action of the tool bodies of said staking machine,
wherein said deformable material of said laminar sample sheet has an extremely low or essentially null elastic memory.

12. Kit according to claim 11 **characterized in that** said laminar sample sheet has an extremely high or substantially maximum value of permanent tensile deformation.

13. Kit according to claim 11 or 12 **characterized in that** said laminar sample sheet is any of the material capable of plastic deformation selected from the group consisting of polymer, crystalline polymer, tar, lead, mineral bound compound and similar.

## Patentansprüche

1. Methode zur Bewertung der Effizienz und/oder des Betriebszustands einer Stollmaschine, speziell für das Gebiet des Gerbens, **dadurch gekennzeichnet, dass** sie die folgenden Operationen umfasst:
- Einführen von mindestens einem laminaren Probenblatt, das aus einem deformierbaren Material hergestellt ist und vorbestimmte Initialdimensionen aufweist, in eine Stollmaschine, die geeignet ist:
• mindestens eine flexible laminare Oberfläche, die zu bearbeiten ist, zu erweichen,
• die flexible laminare Oberfläche zu dehnen oder zu vergrößern;
- Unterwerfen des laminaren Probenblattes der schwingenden oder pulsierenden Aktion der Werkzeugkörper der Stollmaschine;
- Messen der Finaldimensionen des laminaren Probenblattes, wenn es der schwingenden oder pulsierenden Aktion der Werkzeugkörper der Stollmaschine ausgesetzt worden ist;
- Vergleichen der Finaldimensionen mit den Initialdimensionen des laminaren Probenblattes, Berechnen der Effizienz der Stollmaschine durch eine Differenz zwischen den Dimensionen,
wobei das deformierbare Material des laminaren Probenblattes ein extrem geringes oder im Wesentlichen verschwindendes elastisches Gedächtnis aufweist.

2. Methode gemäß Anspruch 1, **dadurch gekennzeichnet, dass** das laminare Probenblatt aus deformierbarem Material hergestellt ist, das einen extrem hohen oder im Wesentlichen maximalen Wert einer permanenten Zugdeformation aufweist.

3. Methode gemäß einem der vorhergehenden Ansprüche, **dadurch gekennzeichnet, dass** das laminare Probenblatt aus deformierbarem Material hergestellt ist, das eine Dicke aufweist, die einen Wert in dem Bereich von 0,013 bis 13,2 mm besitzt.

4. Methode gemäß einem der vorhergehenden Ansprüche, **dadurch gekennzeichnet, dass** das laminare Probenblatt aus deformierbarem Material hergestellt ist, das eine prozentuale Bruchdehnung aufweist, die einen Wert in dem Bereich von 0,3 bis 600 % besitzt.

5. Methode gemäß einem der vorhergehenden Ansprüche, **dadurch gekennzeichnet, dass** das deformierbare Material, mit dem das laminare Probenblatt hergestellt ist, eines der Materialien ist, die zu einer plastischen Deformation in der Lage sind, ausgewählt aus der Gruppe, die aus Polymer, kristallinem Polymer, Teer, Blei, mineral-gebundener Zusammensetzung und ähnlichem besteht.

6. Methode gemäß Anspruch 5, **dadurch gekennzeichnet, dass** das kristalline Polymer Polytetrafluorethylen (PTFE) ist.

7. Verfahren gemäß einem der vorhergehenden Ansprüche, **dadurch gekennzeichnet, dass** die Werkzeugkörper der Stollmaschine die schwingende oder pulsierende Aktion auf dem laminaren Probenblatt bei einem vorgegebenen Druck und mit einer voreingestellten Frequenz von Takten pro Minute ausführen.

8. Methode gemäß Anspruch 7, **dadurch gekennzeichnet, dass** der vorbestimmte Druck und die voreingestellte Frequenz von Takten pro Minute in Abhängigkeit vom Typ der zu bearbeitenden flexiblen laminaren Oberfläche in der Stollmaschine variieren.

9. Methode gemäß einem der vorhergehenden Ansprüche, **dadurch gekennzeichnet, dass** das laminare Probenblatt bei mindestens einem weiteren separaten Zyklus der Bewertung der Effizienz der Stollmaschine wiederverwendet werden kann.

10. Methode gemäß einem der vorhergehenden Ansprüche, **dadurch gekennzeichnet, dass** das laminare Probenblatt gemeinsam mit der zu bearbeitenden flexiblen laminaren Oberfläche in die Stollmaschine eingeführt wird.

11. Kit zur Bewertung der Effizienz und/oder des Betriebszustands einer Stollmaschine, insbesondere für das Gebiet des Gerbens, **dadurch gekennzeichnet, dass** es enthält:
- eine Stollmaschine, die geeignet ist, mindestens eine flexible laminare Oberfläche, die zu bearbeiten ist, zu erweichen und die flexible laminare Oberfläche zu dehnen oder zu vergrößern;
- mindestens ein laminares Probenblatt, das aus deformierbarem Material hergestellt ist und vorbestimmte Initialdimensionen aufweist, geeignet, um in die Stollmaschine eingeführt zu werden, um ähnlich der flexiblen laminaren Oberfläche der schwingenden oder pulsierenden Aktion der Werkzeugkörper der Stollmaschine ausgesetzt zu werden,
wobei das deformierbare Material des laminaren Probenblattes ein extrem geringes oder im Wesentlichen verschwindendes elastisches Gedächtnis aufweist.

12. Kit gemäß Anspruch 11, **dadurch gekennzeichnet, dass** das laminare Probenblatt einen extrem hohen oder im Wesentlichen maximalen Wert der permanenten Zugdeformation aufweist.

13. Kit gemäß Anspruch 11 oder 12, **dadurch gekennzeichnet, dass** das laminare Probenblatt eines der Materialien ist, die zu einer plastischen Deformation in der Lage sind, ausgewählt aus der Gruppe, die aus Polymer, kristallinem Polymer, Teer, Blei, mineral-gebundener Zusammensetzung und ähnlichem besteht.

## Revendications

1. Procédé pour évaluer l'efficacité et/ou l'état de fonctionnement d'une machine de palissonnage, en particulier pour le domaine du tannage, **caractérisé en ce qu'**il comprend les opérations suivantes :
- l'introduction d'au moins une feuille d'échantillon laminaire réalisée en un matériau déformable et ayant des dimensions initiales prédéterminées, dans une machine de palissonnage susceptible de :
• ramollir au moins une surface laminaire souple devant être travaillée,
• étirer ou agrandir ladite surface laminaire souple ;
- le fait de soumettre ladite feuille d'échantillon laminaire à l'action de vibration ou intermittente des corps d'outil de ladite machine de palissonnage ;
- la mesure des dimensions finales de ladite feuille d'échantillon laminaire une fois qu'elle a été soumise à ladite action de vibration ou intermittente desdits corps d'outil de ladite machine de palissonnage ;
- la comparaison desdites dimensions finales auxdites dimensions initiales de ladite feuille d'échantillon laminaire et le calcul de l'efficacité de ladite machine de palissonnage par la différence entre lesdites dimensions,
dans lequel ledit matériau déformable de ladite feuille d'échantillon laminaire a une mémoire élastique extrêmement faible ou essentiellement nulle.

2. Procédé selon la revendication 1, **caractérisé en ce que** ladite feuille d'échantillon laminaire réalisée en un matériau déformable a une valeur de déformation permanente à la traction extrêmement élevée ou sensiblement maximale.

3. Procédé selon l'une quelconque des revendications précédentes, **caractérisé en ce que** ladite feuille d'échantillon laminaire réalisée en un matériau déformable a une épaisseur ayant une valeur dans la plage de 0,013 ÷ 13,2 mm.

4. Procédé selon l'une quelconque des revendications précédentes, **caractérisé en ce que** ladite feuille d'échantillon laminaire réalisée en un matériau déformable a un pourcentage d'allongement ayant une valeur dans la plage de 0,3 ÷ 600%.

5. Procédé selon l'une quelconque des revendications précédentes, **caractérisé en ce que** ledit matériau déformable avec lequel est réalisée ladite feuille d'échantillon laminaire est l'un quelconque des matériaux susceptibles de subir une déformation plastique sélectionné parmi le groupe comprenant un polymère, un polymère cristallin, le goudron, le plomb, un composé lié minéral, et similaire.

6. Procédé selon la revendication 5, **caractérisé en ce que** ledit polymère polycristallin est du polytétrafluoroéthylène (PTFE).

7. Procédé selon l'une quelconque des revendications précédentes, **caractérisé en ce que** lesdits corps d'outil de ladite machine de palissonnage effectuent ladite action de vibration ou intermittente sur ladite feuille d'échantillon laminaire à une pression prédéfinie et avec une fréquence pré-fixée de coups par minutes.

8. Procédé selon la revendication 7, **caractérisé en ce que** ladite pression prédéfinie et ladite fréquence pré-fixée de coups par minute varient en fonction du type de ladite surface laminaire souple devant être travaillée dans ladite machine de palissonnage.

9. Procédé selon l'une quelconque des revendications précédentes, **caractérisé en ce que** ladite feuille d'échantillon laminaire peut être réutilisée dans au moins un autre cycle d'évaluation séparé de ladite efficacité de ladite machine de palissonnage.

10. Procédé selon l'une quelconque des revendications précédentes, **caractérisé en ce que** ladite feuille d'échantillon laminaire est introduite dans ladite machine de palissonnage avec ladite surface laminaire souple devant être travaillée.

11. Ensemble pour l'évaluation de l'efficacité et/ou de l'état de fonctionnement d'une machine de palissonnage, en particulier pour le secteur du tannage, **caractérisé en ce qu'**il comprend :
- une machine de palissonnage appropriée pour ramollir au moins une surface laminaire souple devant être travaillée et étirer ou agrandir ladite surface laminaire souple ;
- au moins une feuille d'échantillon laminaire, réalisée en un matériau déformable et ayant des dimensions initiales prédéterminées, appropriée pour être introduite dans ladite machine de palissonnage de façon à être soumise, de façon similaire à ladite surface laminaire souple, à l'action de vibration ou intermittente des corps d'outil de ladite machine de palissonnage,
dans lequel ledit matériau déformable de ladite feuille d'échantillon laminaire a une mémoire élastique extrêmement faible ou essentiellement nulle.

12. Ensemble selon la revendication 11, **caractérisé en ce que** ladite feuille d'échantillon laminaire a une valeur de déformation permanente à la traction extrêmement élevée ou sensiblement maximale.

13. Ensemble selon la revendication 11 ou 12, **caractérisé en ce que** ladite feuille d'échantillon laminaire est l'un quelconque du matériau susceptible de subir une déformation plastique sélectionné parmi le groupe comprenant un polymère, un polymère cristallin, le goudron, le plomb, un composé lié minéral, et similaire.
